# EUROPEAN PATENT APPLICATION

(11) **EP 4 763 038 A1**
(43) Date of publication of application: **24.06.2026**
(21) Application number: 24220647.2
(22) Date of filing: 17.12.2024
(51) Int. Cl.: A61B 1/00, A61B 1/045, A61B 1/06, A61B 1/24, A61B 5/00

(54) **CONTROLLING IMAGING MODALITY**

(71) Applicant: Koninklijke Philips N.V., 5656 AE Eindhoven (NL)
(72) Inventor: VLUTTERS, Ruud, Eindhoven (NL); GALLUCCI, Alessio, Eindhoven (NL)
(74) Representative: Philips Intellectual Property & Standards

(57) **Abstract**

Proposed concepts thus aim to provide schemes, solutions, concepts, designs, methods and systems pertaining to controlling imaging modality of an oral imaging device. In particular, embodiments aim to provide a method for controlling imaging modality of an oral imaging device by basing the decision to switch imaging modality on whether there is an oral health feature presently or soon to be in view of the camera. In other words, it is proposed that by determining whether there is an oral health feature (e.g., a cavity) in view of the camera of the oral imaging device, the oral imaging device can automatically be put in the optimal available imaging modality for imaging said oral health feature.

## Description

### FIELD OF THE INVENTION

This invention relates to the field of controlling imaging modality of an oral imaging device.

### BACKGROUND OF THE INVENTION

Oral imaging cameras are becoming increasingly common, for instance, to detect plaque, but they often produce low-quality images which are difficult to work with. For example, when using white light with a standard RGB colour camera, plaque is often difficult to identify. Plaque is easier to identify if using quantitative light-induced fluorescence (QLF), however, gums are typically not visible in this imaging modality and thus gum issues cannot be detected.

Some oral imaging cameras are capable of capturing images in more than one imaging modality (e.g., white light, QLF, infrared light, change of aperture, etc.), however a user often will not know which imaging modality to use for different parts of their mouth. It is also not an optimal solution to capture images of the entire mouth in all imaging modalities as this is not time or energy efficient.

### SUMMARY OF THE INVENTION

The invention is defined by the claims.

According to examples in accordance with an aspect of the invention, there is provided a computer-implemented method for controlling imaging modality of an oral imaging device, the method comprising: determining the presence of an oral health feature in a subject's mouth in view of a camera of the oral imaging device; and controlling the oral imaging device to switch from a first imaging modality to a second imaging modality based on the determined presence of an oral health feature.

Proposed concepts thus aim to provide schemes, solutions, concepts, designs, methods and systems pertaining to controlling imaging modality of an oral imaging device. In particular, embodiments aim to provide a method for controlling imaging modality of an oral imaging device by basing the decision to switch imaging modality on whether there is an oral health feature in view of the camera.

In other words, it is proposed that by determining whether there is an oral health feature (e.g., a cavity) in view of the camera of the oral imaging device, the oral imaging device can automatically be put in the optimal available imaging modality for imaging said oral health feature. In this way, a subject's mouth can be imaged in an effective and efficient way, with each part of the subject's mouth being captured using the most useful available imaging modality for the oral health feature(s) present in each part of the subject's mouth (i.e., without having to capture images of the subject's entire mouth in multiple imaging modalities).

In summary, the oral imaging device is controlled to switch imaging modality dependent on what oral health feature(s) are in view (or about to be in view) of the camera of the oral imaging device.

Ultimately, an improved method for controlling imaging modality of an oral imaging device is provided.

In some embodiments, determining the presence of an oral health feature may comprise analysing an image captured by the camera of the oral imaging device to detect the oral health feature. In this way, an efficient use of the oral imaging device may be provided (as images will typically already be taken by the oral imaging device), allowing the presence of oral health features to be directly determined without requiring any extra information or an additional device.

In some embodiments, determining the presence of an oral health feature may comprise: capturing an image of at least one oral feature of the subject using the camera of the oral imaging device, wherein an oral feature comprises a portion of at least one of: a tooth; gum; tongue; and a lip; analysing the captured image to determine the location of the camera; and determining the presence of an oral health feature in view of the camera based on the determined location and oral health data describing the presence and location of at least one oral health feature in the mouth of the subject. In this way, the presence of an oral health feature may not need to be directly determined by analysing an image (i.e., identifying the oral health feature itself in the image) but rather only the location of the camera needs to be determined and then prior/historical oral health data (e.g., describing that a subject's molar is known to have a filling) can be used to infer the presence of an oral health feature in view of the camera.

In some embodiments, the method may further comprise: predicting a future location of the camera at a future time point based on a motion of the camera and a present location of the camera; determining the presence of an oral health feature at the predicted future location of the camera based on the predicted future location and oral health data describing the presence and location of at least one oral health feature in the mouth of the subject; and controlling the oral imaging device to switch from a first imaging modality to a second imaging modality based on the determined presence of an oral health feature at the predicted future location of the camera. In this way, the oral imaging device may be controlled based on what oral health feature will be in view of the camera at a future time point.

In some embodiments, controlling the oral imaging device may comprise controlling the oral imaging device to switch from a first imaging modality to a second imaging modality such that the oral imaging device will be in the second imaging modality at the future time point. In this way, use of the oral imaging device may be made more efficient such that the oral imaging device may essentially pre-emptively switch imaging modality so that it is in the correct imaging modality by the time it reaches a location where it is known that a particular oral health feature is present. A user may then not have to wait once they reach said location for the oral imaging device to switch imaging modality.

In some embodiments, the method may further comprise determining a motion of the camera based on data from an inertial measurement sensor associated with the oral imaging device. This may provide an effective way to obtain the motion of the camera.

In some embodiments, the method may further comprise determining a motion of the camera based on a captured series of images of at least one oral feature of the subject, wherein an oral feature comprises a portion of at least one of: a tooth; gum; tongue; and a lip. This may allow the motion of the camera to be obtained without the need for additional equipment.

In some embodiments, the oral health data may further describe the presence and location of at least one predicted oral health feature in the mouth of the subject. This may thus allow the oral imaging device to be controlled based on oral health features which are not 'known' to be present at a particular location in the subject's mouth but are predicted to be. For example, it may be known that if a subject has plaque on tooth 13, it is highly likely that they will also have plaque on tooth 14. The prediction of there being plaque present on tooth 14 can thus be used to control the oral imaging device (i.e., to select the imaging modality for imaging tooth 14).

In some embodiments, determining the presence of an oral health feature may be further based on a demographic parameter of the subject, wherein a demographic parameter comprises at least one of: age; gender; race; ethnicity; weight; height; BMI; and geographic location. This may allow the presence of oral health features to be more accurately determined, taking into account, for example, the probabilities of various oral health features being present depending on the subject's demographics.

In some embodiments, an oral health feature may comprise at least one of: a cavity; a crown; a bridge; a filling; plaque; tartar; gingivitis; periodontitis; tooth erosion; an abscessed tooth; malocclusion; a denture; a dental implant; braces; an impacted tooth; and a tumor. These may each be useful oral health features to determine the presence of and thus allow a beneficial imaging modality to be chosen for imaging said feature.

In some embodiments, the second imaging modality may comprise at least one of: white light; quantitative light-induced fluorescence; fluorescence imaging with reflectance enhancement; and infrared light. These may be particularly useful imaging modalities for imaging various different oral health features.

In some embodiments, the method may further comprise selecting the second imaging modality from a plurality of available imaging modalities based on the type of oral health feature determined to be present. In this way, if the oral imaging device has three or more imaging modalities, the most beneficial for the type of oral health feature to-be-imaged can be switched to.

In some embodiments, determining the presence of an oral health feature may comprise using a machine-learning model trained to determine the presence of an oral health feature based on at least one image captured by the camera of the oral imaging device. A machine-learning model may provide a particularly effective way to determine the presence of an oral health feature directly from an image.

According to another aspect of the invention, there is provided a computer program comprising code means for implementing the method of any herein disclosed method when said program is run on a processor arrangement.

According to another aspect of the invention, there is provided a system for controlling imaging modality of an oral imaging device, comprising: a processor arrangement configured to: determine the presence of an oral health feature in a subject's mouth in view of a camera of the oral imaging device; and control the oral imaging device to switch from a first imaging modality to a second imaging modality based on the determined presence of an oral health feature.

Thus, there may be proposed concepts for controlling imaging modality of an oral imaging device, and this may be done based on determining the presence of an oral health feature in view of the camera.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Fig. 1 is a simplified flow diagram of a method for controlling imaging modality of an oral imaging device according to a proposed embodiment;
Fig. 2 is a flow diagram of a method for controlling imaging modality of an oral imaging device comprising analysing an image according to a proposed embodiment;
Fig. 3 is a flow diagram of a method for controlling imaging modality of an oral imaging device comprising determining a location of the camera according to a proposed embodiment;
Fig. 4 is a flow diagram of a method for controlling imaging modality of an oral imaging device comprising predicting a future location of the camera according to a proposed embodiment;
Fig. 5 is a simplified block diagram of a system for controlling imaging modality of an oral imaging device according to a proposed embodiment; and
Fig. 6 illustrates an example of a computer within which one or more parts of an embodiment may be employed.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

Implementations in accordance with the present disclosure relate to various techniques, methods, schemes and/or solutions pertaining to controlling imaging modality of an oral imaging device. According to proposed concepts, a number of possible solutions may be implemented separately or jointly. That is, although these possible solutions may be described below separately, two or more of these possible solutions may be implemented in one combination or another.

Embodiments of the invention aim to provide a method for controlling imaging modality of an oral imaging device. This can be achieved by basing the decision to switch imaging modality on whether there is an oral health feature currently (or soon to be) in view of the camera.

In other words, it is proposed that by determining whether there is an oral health feature (e.g., a cavity) in view of the camera of the oral imaging device (either in the present or at a predicted future time point), the oral imaging device can automatically be put in the optimal available imaging modality for imaging said oral health feature. In this way, a subject's mouth can be imaged in an effective and efficient way, with each part of the subject's mouth being captured using the most useful available imaging modality for the oral health feature(s) present in each part of the subject's mouth (i.e., without having to capture images of the subject's entire mouth in multiple imaging modalities).

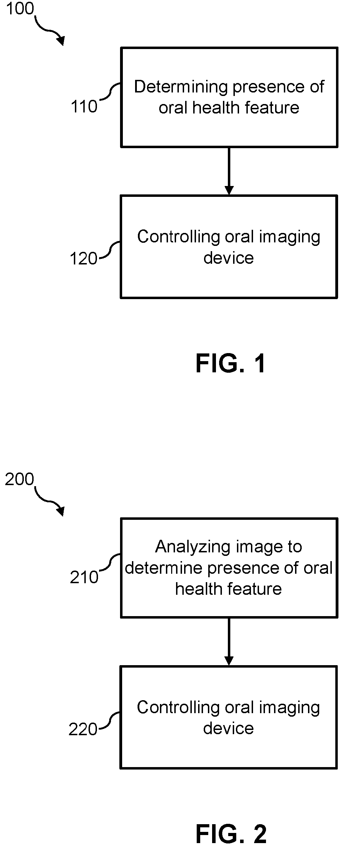

Referring now to Fig. 1, there is depicted a simplified flow diagram of a method 100 for controlling imaging modality of an oral imaging device according to a proposed embodiment.

The method 100 begins with the step 110 of determining the presence of an oral health feature in a subject's mouth in view of a camera of the oral imaging device. For example, in some embodiments, the oral imaging device can comprise a plurality of cameras - step 110 would thus comprise determining that an oral health feature is present in view of at least one of the plurality of cameras of the oral imaging device. It should also be noted that in view of a camera should not be understood as immediately identifiable by said camera. For instance, the camera in a first imaging modality may not be able to actually see the oral health feature even though it is looking at it - this would still count as in view of the camera as in another imaging modality, the oral health feature would be identifiable by said camera without the camera having to move. It should also be noted that an oral imaging device is to be understood as a device which images a subject's mouth from within their mouth, i.e., intraoral. This does not mean that the entire device must be within the subject's mouth, only at least a camera of the oral imaging device.

It should also be noted that determining the presence of an oral health feature in view of the camera is not restricted to the present (i.e., what is currently in view of the camera) but also extends to oral health features which are predicted to be in view of the camera at a future time point. Step 110 can thus alternatively be understood as determining the presence of an oral health feature in a subject's mouth currently in view of a camera of the oral imaging device or predicted to be in view of the camera at a future time point.

It should also be noted that determining the presence of an oral health feature does not mean that it is determined that the oral health feature is 100% definitively present, for instance, it can be predicted that the oral health feature is present but not certainly known - this would still count as determining the presence of an oral health feature. In some embodiments, an oral health feature may only be determined to be present if the certainty of the prediction is above a predetermined threshold (e.g., 70% / 80% / 90%, etc.).

In this embodiment, an oral health feature comprises at least one of: a cavity; a crown; a bridge; a filling; plaque; tartar; gingivitis; periodontitis; tooth erosion; an abscessed tooth; malocclusion; a denture; a dental implant; braces; an impacted tooth; and a tumor. These are each useful oral health features to determine the presence of and thus allow a beneficial imaging modality to be chosen for imaging said feature. In other embodiments, however, an oral health feature can comprise any suitable type of oral health feature (i.e., any type of feature/artefact relevant to oral health).

In some embodiments, determining the presence of an oral health feature in step 110 can further be based on a demographic parameter of the subject, wherein a demographic parameter comprises at least one of: age; gender; race; ethnicity; weight; height; BMI; and geographic location. This can allow the presence of oral health features to be more accurately determined, taking into account, for example, the probabilities of various oral health features being present depending on the subject's demographics. For instance, older subjects could be more likely to develop periodontitis, and subjects above a certain weight could be more likely to develop cavities. It should be noted that in other embodiments, the demographic parameter can comprise any suitable parameter which may influence the probabilities of various oral health features being present. In addition, in some embodiments, demographic parameters of a subject may be used to help estimate a 3D head model / jaw model of the subject which may then be used to help determine the presence of an oral health feature.

It should be noted that in some embodiments, step 110 can comprise not only determining the presence of an oral health feature but also identifying/determining the type of oral health feature.

The method 100 also comprises step 120 of controlling the oral imaging device to switch from a first imaging modality to a second imaging modality based on the determined presence of an oral health feature. For example, if a particular oral health feature is determined to be present in view of the camera, it could be preferred that the second imaging modality is used to image it, and thus the oral imaging device would be controlled to switch from a first imaging modality to the second. Of course, the oral imaging device could already be in the optimal available imaging modality for imaging the oral health feature and in this case, the oral imaging device would be controlled not to switch imaging modality but to stay in the imaging modality it is already in. In other words, step 120 could alternatively be understood as: controlling the imaging modality of the oral imaging device based on the determined presence of an oral health feature, wherein the oral imaging device has at least two imaging modalities.

In this embodiment, the second imaging modality (and the first imaging modality) comprises at least one of: white light; quantitative light-induced fluorescence; fluorescence imaging with reflectance enhancement; and infrared light. The first and second imaging modalities are always different from one another. For example, if the first imaging modality was white light, the second imaging modality could be quantitative light-induced fluorescence, fluorescence imaging with reflectance enhancement, or infrared light.

White light imaging modality can be understood as capturing an image while at least the portion of the subject's mouth in view of the camera is illuminated with white light. This is particularly useful for making a subject's teeth and gums visible.

Similarly, infrared imaging modality can be understood as capturing an image while at least the portion of the subject's mouth in view of the camera is illuminated with infrared light. This is particularly useful for visualizing cavities (i.e., caries).

Quantitative light-induced fluorescence (QLF) imaging modality can be understood as capturing an image while at least the portion of the subject's mouth in view of the camera is illuminated with blue light (e.g., light with a wavelength between 400 and 500nm, and preferably around 405nm). This results in plaque and tartar being more visible (than when using white light) through red self-fluorescence (while teeth are visible in green), though gums are not visible / poorly visible.

When QLF is being used, it is preferable to use a band pass filter in front of the camera to block blue excitation light, though this is not essential. In some embodiments, a band pass filter can be provided which is mechanically positionable, such that when the oral imaging device is in QLF imaging modality, the band pass filter can be automatically moved in front of the camera, and when the oral imaging device is in another imaging modality, the band pass filter can be moved away from the camera (i.e., out of view of the camera).

Fluorescence imaging with reflectance enhancement (FIRE) imaging modality can be understood as similar to QLF imaging, however, with a portion of white light also used to illuminate the portion of the subject's mouth in view of the camera (in addition to the blue light). This allows the gums to also be seen.

Of course, in other embodiments, as the skilled person would appreciate, the first and second imaging modalities can comprise any suitable imaging modalities for imaging the mouth of a subject and oral health features, such as, for example, polarized light or ultraviolet. Alternatively, or in addition, to using different lighting while capturing an image, two image modalities can also be different by virtue of different camera settings (e.g., a first image modality using white light with a first exposure/contrast setting and a second image modality also using white light but with a different second exposure/contrast setting). For instance, a camera setting which might be changed between different imaging modalities could comprise at least one of: field of view; shutter speed; aperture; exposure; contrast; and an intraoral scanner (IOS) distance spacer to mechanically force a specific distance between the camera and the subject of the image. In some embodiments, each imaging modality can include a lighting mode (e.g., white light, QLF, FIRE, or infrared) and an associated set of camera settings (e.g., field of view, shutter speed, aperture, etc.).

In this embodiment, the oral imaging device only comprises two imaging modalities (the first and second imaging modalities), however, in other embodiments the oral imaging device can comprise three or more imaging modalities. In these embodiments, the method 100 can further comprise selecting the second imaging modality (i.e., the imaging modality being switched to) from a plurality of available imaging modalities (not including the first imaging modality) based on the type of oral health feature determined to be present. In this way, the most beneficial imaging modality for the type of oral health feature to-be-imaged can be switched to. It should be noted that in some embodiments, multiple imaging modalities may be determined to be equally beneficial (or beneficial in combination) for the same oral health feature, and thus in these cases, the second imaging modality can be selected to be one of these beneficial imaging modalities and then the method repeated with the other beneficial imaging modality(s) then selected as the second imaging modality (and so on). Images of the same oral health feature captured in multiple imaging modalities may increase the prediction accuracy of some specific types of oral health features.

In some embodiments, once the oral imaging device is in the correct imaging modality, multiple images can be captured per location, e.g., with various different contrast settings, in order to maximize information intake. This would, however, take longer and so could be used on a 'slower' setting of the oral imaging device and ignored on a 'faster' setting where, for example, only one image is captured per location.

In summary, the present invention provides smart scanning of a subject's mouth with the oral imaging device adaptively changing its scanning protocol (i.e., its imaging modality) based on oral artifacts (i.e., oral health features) present in the subject's mouth. For example, the oral imaging device can be controlled to automatically switch to QLF imaging when plaque has been detected in front of the camera, e.g., using a plaque detection algorithm, (or plaque is known to be at the camera's location, as described later in relation to method 300). Later on, for example, the oral imaging device could be controlled to automatically switch to FIRE imaging when cavities are detected (or are known to be present).

Referring now to Fig. 2, there is depicted a diagram of a method 200 for controlling imaging modality of an oral imaging device comprising analysing an image according to a proposed embodiment. Step 220 is substantially the same as step 120 as described in relation to method 100 of Fig. 1.

Determining the presence of an oral health feature in method 200 comprises step 210 of analysing an image captured by a camera of the oral imaging device to detect the oral health feature. In this way, an efficient use of the oral imaging device is provided (as images will typically already be taken by the oral imaging device), allowing the presence of oral health features to be directly determined without requiring any extra information or an additional device.

In some embodiments, a machine-learning model trained to determine the presence of (i.e., detect) an oral health feature based on at least one image captured by the camera of the oral imaging device can be used to perform step 210. In other words, in some embodiments, step 210 can comprise using a trained machine-learning model to analyse an image and detect (and identify) an oral health feature within said image. This is not essential, however, and in other embodiments, any suitable method for analysing an image to determine the presence of (and optionally, identify the type of) an oral health feature can be used, as would be understood by the skilled person.

As the skilled person would appreciate, in some embodiments, the machine-learning model can comprise a trained artificial neural network. It should also be noted that machine-learning models or artificial neural networks can also be used for other suitable tasks disclosed herein, such as determining the location of the camera based on image(s) captured by said camera.

In a specific example of the invention, there is provided a computer-implemented method comprising: obtaining an input image captured by the intra-oral camera of the oral imaging device; determining a current location of the intra-oral camera relative to the subject's mouth based on the input image (e.g., using an AI model); feeding the input image and the determined location of the camera to one or more trained machine-learning models to predict the probabilities of one or more oral conditions (i.e., oral health features) currently present in view of the camera; determining which oral conditions are present in the current frame of the camera based on the predicted probabilities (e.g., oral health features which are predicted to be present with a probability above 80/90/95% are determined as present), as well as optionally predicting which oral conditions will be present in the next frame of the camera (i.e., at an immediate future location of the camera); and, based on the oral health features determined to be present in the current frame, accordingly controlling the oral imaging device to switch to the best imaging modality for the most probable oral condition present in view of the camera (and then to switch to the best imaging modality for the most probable oral condition predicted to be present in the next frame of the camera). In further examples, not only can the input image and determined location be fed into the one or more trained machine-learning models to predict the probabilities of one or more oral conditions, but a change in location/speed of the intra-oral camera over time, and demographic/population statistics relevant to oral conditions can also be fed into the one or more models to make the prediction more accurate.

It is noted that determining which oral conditions are present in the current frame of the camera based on the predicted probabilities can be enacted in a variety of ways, as would be understood by the skilled person. For example, predetermined absolute thresholds can be used, as described in the example above (e.g., above 80%). Alternatively, relative thresholds can be used, for example, when using a multiclass model with several classes, a probability for the presence of a certain oral condition may be 40% while all the probabilities for the other classes are much lower, e.g., less then 5% - in this case, the 40% could be interpreted as a sufficiently (relatively) strong signal and the feature determined as present. In another example, for instance, when the prevalence of a particular oral condition is low or difficult to predict, a lower absolute probability threshold for that oral condition in particular may be used, for example, only 60% while the other oral conditions require a probability of 70% for said condition to be determined as present.

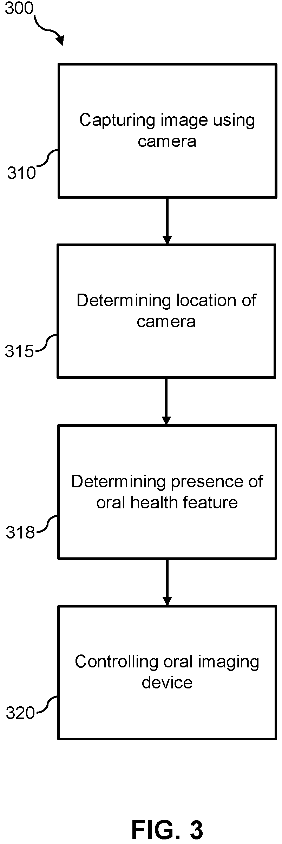

Referring now to Fig. 3, there is depicted a flow diagram of a method 300 for controlling imaging modality of an oral imaging device comprising determining a location of the camera according to a proposed embodiment. Step 320 is substantially the same as step 120 described in relation to method 100 of Fig. 1.

Step 310 comprises capturing an image of at least one oral feature of the subject using the camera of the oral imaging device. An oral feature comprises a portion (i.e., part or whole) of at least one of: a tooth; gum; tongue; and a lip. To be clear, an oral feature is a separate (broader) classification of features to an oral health feature. Whereas oral health features are concerned with specific features related to oral health (e.g., plaque or cavities), an oral feature is merely any feature/object within a subject's mouth (e.g., tooth, gum, tongue, lip, etc.) An oral feature may thus also be referred to as an oral object or a mouth feature/object. For instance, an image could be captured in step 310 of one tooth of the subject, or of two teeth of the subject, or of the tongue of the subject, or a portion of the subject's gum, etc. As described above, in some embodiments, the oral imaging device can comprise more than one camera, and in these embodiments, step 310 would be understood as capturing an image of at least one tooth or the gum of the subject using at least one camera of the oral imaging device.

Step 315 comprises analysing the captured image to determine the location of the camera (at the time at which the image was captured, of course). This can be performed in any suitable way, as would be understood by the skilled person, for example, using a trained machine-learning model or AI. Step 318 comprises determining the presence of an oral health feature in view of the camera based on the determined location (determined in step 315) and oral health data describing the presence and location of at least one oral health feature in the mouth of the subject. For example, the oral health data could take the form of a database of oral health features known to be in the mouth of the subject (e.g., from prior imaging of the subject's mouth (e.g., using the same oral imaging device, essentially internally building up said oral health data) or dental records or any other suitable prior/historical information) and their respective locations. As the skilled person would understand, however, the oral health data need not take the form of a database and could take any suitable form for referencing. In this way, the presence of an oral health feature does not need to be directly determined by analysing an image (i.e., identifying the oral health feature itself in the image) but rather only the location of the camera needs to be determined and then prior/historical oral health data (e.g., describing that a subject's molar is known to have a filling) can be used to infer the presence of an oral health feature in view of the camera.

In some embodiments, the oral health data can also further describe the presence and location of at least one predicted oral health feature in the mouth of the subject. This would thus allow the oral imaging device to be controlled based on oral health features which are not 'known' to be present at a particular location in the subject's mouth but are predicted to be. For example, it may be known that if a subject has plaque on tooth 13, it is highly likely that they will also have plaque on tooth 14. The prediction of there being plaque present on tooth 14 can thus be used to control the oral imaging device (i.e., to select the imaging modality for imaging tooth 14). In this way, not only can prior knowledge be used to help determine when to switch imaging modality but also extrapolations of said prior knowledge can be used to help determine when to switch imaging modality. Predicting oral health features can also make use of demographic parameters in much the same way as was described in relation to step 110.

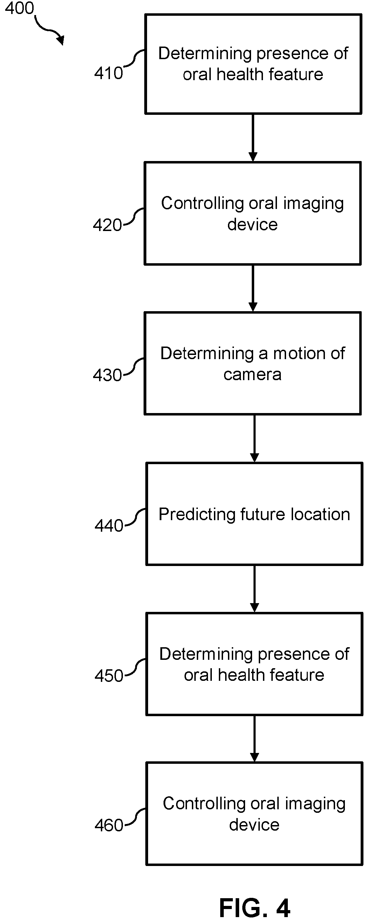

Referring now to Fig. 4, there is depicted a flow diagram of a method 400 for controlling imaging modality of an oral imaging device comprising predicting a future location of the camera according to a proposed embodiment. Steps 410 and 420 are substantially the same as steps 110 and 120 respectively as described in relation to method 100 of Fig. 1.

Step 430 comprises determining a motion of the camera. This can be done in many ways, as the skilled person would understand. For example, determining a motion of the camera could be based on data from an inertial measurement sensor associated with (i.e., within or attached to) the oral imaging device. This provides an effective way to obtain the motion of the camera. In another example, determining a motion of the camera could be based on a captured series of images of at least one oral feature of the subject (for example, by using an object detector to detect (bounding box) and segment (mask) each individual tooth, such that by tracking the boundary boxes over the video frames, motion of the camera can be determined). As described above, an oral feature comprises a portion (i.e., part or whole) of at least one of: a tooth; gum; tongue; and a lip. This would allow the motion of the camera to be obtained without the need for additional equipment (i.e., purely based on analysis of the distance moved by the camera between the images of the captured series of images). In yet another example, to enhance reliability and/or accuracy of the determined motion, the motion of the camera could be determined based on data from an inertial measurement sensor associated with the oral imaging device and a captured series of images of at least one oral feature of the subject.

In other embodiments, step 430 could be replaced and the motion of the camera could be obtained in any other suitable way, e.g., without having to directly determine it, such as, for example, obtaining the motion of the camera from an external device/system.

Step 440 comprises predicting a future location of the camera at a future time point (i.e., time location/stamp) based on the motion of the camera and a present location of the camera.

As the skilled person would understand, the present location of the camera can be obtained in many different suitable ways. For example, the present location of the camera could be determined in a manner similar to that of step 315 of method 300.

Step 450 comprises determining the presence of an oral health feature at the predicted future location of the camera based on the predicted future location and oral health data describing the presence and location of at least one oral health feature in the mouth of the subject. The oral health data is substantially the same as the oral health data described in relation to step 318 of method 300 (and as described above, could also comprise the presence and location of at least one predicted oral health feature too).

Step 460 comprises controlling the oral imaging device to switch from a first imaging modality to a second imaging modality based on the determined presence of an oral health feature at the predicted future location of the camera. In this way, the oral imaging device can be controlled based on what oral health feature will soon be in view of the camera.

For instance, in this embodiment, controlling the oral imaging device in step 460 specifically comprises controlling the oral imaging device to switch from a first imaging modality to a second imaging modality such that the oral imaging device will be in the second imaging modality at the future time point. In this way, use of the oral imaging device is made more efficient such that the oral imaging device can essentially pre-emptively switch imaging modality so that it is in the correct imaging modality by the time it reaches a location where it is known (or predicted) that a particular oral health feature is present. Of course, to ensure this is achieved, it must be known how long it takes for the oral imaging device to switch imaging modality (or the number of frames lost when switching modality) - this could be obtained as prior knowledge. A user will then not have to wait once they reach said location for the oral imaging device to switch imaging modality.

It should be noted that in some embodiments, the method 400 could comprise only steps 430 to 460, i.e., only switch imaging modality based on an oral health feature determined to be in view of the camera at a predicted future location of the camera, such that the oral imaging device has always switched imaging modality before the camera reaches said predicted future location. In other words, in some embodiments, the oral imaging device could be controlled to always pre-emptively switch in anticipation of a determined oral health feature being in view of the camera at a future predicted location.

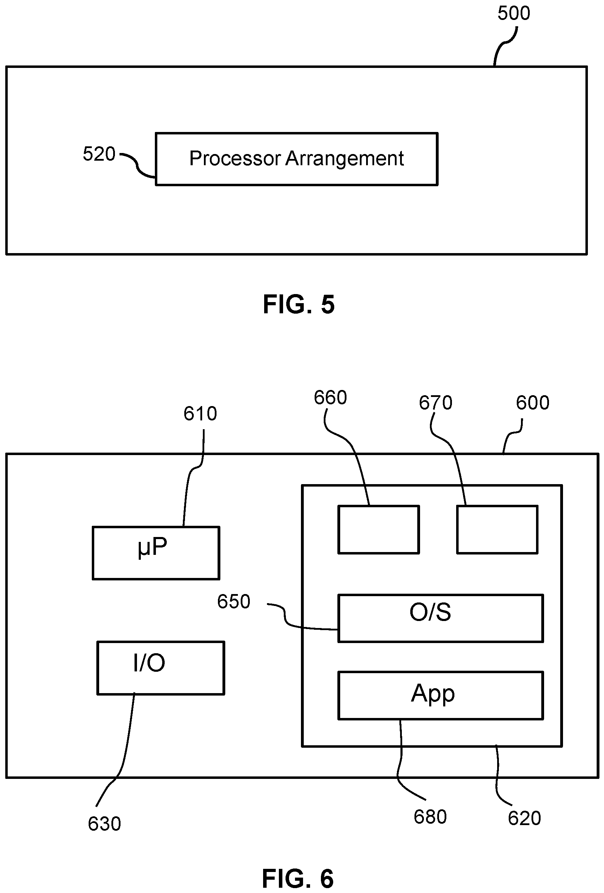

Referring now to Fig. 5, there is depicted a system 500 for controlling imaging modality of an oral imaging device according to a proposed embodiment. The system 500 comprises a processor arrangement 520.

The processor arrangement 520 is configured to perform any of the methods disclosed herein (e.g., method 100, 200, 300, or 400). For example, in this embodiment, the processor arrangement 520 is configured to perform method 100, i.e., to: determine the presence of an oral health feature in a subject's mouth in view of a camera of the oral imaging device; and control the oral imaging device to switch from a first imaging modality to a second imaging modality based on the determined presence of an oral health feature.

In some embodiments, the system 500 can comprise part of the oral imaging device itself, the oral imaging device being a device capable of capturing an image in at least two imaging modalities and comprising at least one camera. In other embodiments, the system 500 can be external to the oral imaging device and communicate with it (e.g., obtain images captured by it and issue controlling instructions) either with wires or wirelessly.

Fig. 6 illustrates an example of a computer 600 within which one or more parts of an embodiment may be employed. Various operations discussed above may utilize the capabilities of the computer 600. In this regard, it is to be understood that system functional blocks can run on a single computer or may be distributed over several computers and locations (e.g. connected via internet).

The computer 600 includes, but is not limited to, PCs, workstations, laptops, PDAs, palm devices, servers, storages, and the like. Generally, in terms of hardware architecture, the computer 600 may include one or more processors 610, memory 620 and one or more I/O devices 630 that are communicatively coupled via a local interface (not shown). The local interface can be, for example but not limited to, one or more buses or other wired or wireless connections, as is known in the art. The local interface may have additional elements, such as controllers, buffers (caches), drivers, repeaters, and receivers, to enable communications. Further, the local interface may include address, control, and/or data connections to enable appropriate communications among the aforementioned components.

The processor 610 is a hardware device for executing software that can be stored in the memory 620. The processor 610 can be virtually any custom made or commercially available processor, a central processing unit (CPU), a digital signal processor (DSP), or an auxiliary processor among several processors associated with the computer 600, and the processor 610 may be a semiconductor based microprocessor (in the form of a microchip) or a microprocessor.

The memory 620 can include any one or combination of volatile memory elements (e.g., random access memory (RAM), such as dynamic random access memory (DRAM), static random access memory (SRAM), etc.) and non-volatile memory elements (e.g., ROM, erasable programmable read only memory (EPROM), electronically erasable programmable read only memory (EEPROM), programmable read only memory (PROM), tape, compact disc read only memory (CD-ROM), disk, diskette, cartridge, cassette or the like, etc.). Moreover, the memory 620 may incorporate electronic, magnetic, optical, and/or other types of storage media. Note that the memory 620 can have a distributed architecture, where various components are situated remote from one another, but can be accessed by the processor 610.

The software in the memory 620 may include one or more separate programs, each of which comprises an ordered listing of executable instructions for implementing logical functions. The software in the memory 620 includes a suitable operating system (O/S) 650, compiler 660, source code 670, and one or more applications 680 in accordance with exemplary embodiments. As illustrated, the application 680 comprises numerous functional components for implementing the features and operations of the exemplary embodiments. The application 680 of the computer 600 may represent various applications, computational units, logic, functional units, processes, operations, virtual entities, and/or modules in accordance with exemplary embodiments, but the application 680 is not meant to be a limitation.

The operating system 650 controls the execution of other computer programs, and provides scheduling, input-output control, file and data management, memory management, and communication control and related services. It is contemplated by the inventors that the application 680 for implementing exemplary embodiments may be applicable on all commercially available operating systems.

Application 680 may be a source program, executable program (object code), script, or any other entity comprising a set of instructions to be performed. When a source program, then the program is usually translated via a compiler (such as the compiler 660), assembler, interpreter, or the like, which may or may not be included within the memory 620, so as to operate properly in connection with the O/S 650. Furthermore, the application 680 can be written as an object oriented programming language, which has classes of data and methods, or a procedure programming language, which has routines, subroutines, and/or functions, for example but not limited to, C, C++, C#, Pascal, Python, BASIC, API calls, HTML, XHTML, XML, ASP scripts, JavaScript, FORTRAN, COBOL, Perl, Java, ADA, .NET, and the like.

The I/O devices 630 may include input devices such as, for example but not limited to, a mouse, keyboard, scanner, microphone, camera, etc. Furthermore, the I/O devices 630 may also include output devices, for example but not limited to a printer, display, etc. Finally, the I/O devices 630 may further include devices that communicate both inputs and outputs, for instance but not limited to, a NIC or modulator/demodulator (for accessing remote devices, other files, devices, systems, or a network), a radio frequency (RF) or other transceiver, a telephonic interface, a bridge, a router, etc. The I/O devices 630 also include components for communicating over various networks, such as the Internet or intranet.

If the computer 600 is a PC, workstation, intelligent device or the like, the software in the memory 620 may further include a basic input output system (BIOS) (omitted for simplicity). The BIOS is a set of essential software routines that initialize and test hardware at start-up, start the O/S 650, and support the transfer of data among the hardware devices. The BIOS is stored in some type of read-only-memory, such as ROM, PROM, EPROM, EEPROM or the like, so that the BIOS can be executed when the computer 600 is activated.

When the computer 600 is in operation, the processor 610 is configured to execute software stored within the memory 620, to communicate data to and from the memory 620, and to generally control operations of the computer 600 pursuant to the software. The application 680 and the O/S 650 are read, in whole or in part, by the processor 610, perhaps buffered within the processor 610, and then executed.

When the application 680 is implemented in software it should be noted that the application 680 can be stored on virtually any computer readable medium for use by or in connection with any computer related system or method. In the context of this document, a computer readable medium may be an electronic, magnetic, optical, or other physical device or means that can contain or store a computer program for use by or in connection with a computer related system or method.

The application 680 can be embodied in any computer-readable medium for use by or in connection with an instruction execution system, apparatus, or device, such as a computer-based system, processor-containing system, or other system that can fetch the instructions from the instruction execution system, apparatus, or device and execute the instructions. In the context of this document, a "computer-readable medium" can be any means that can store, communicate, propagate, or transport the program for use by or in connection with the instruction execution system, apparatus, or device. The computer readable medium can be, for example but not limited to, an electronic, magnetic, optical, electromagnetic, infrared, or semiconductor system, apparatus, device, or propagation medium.

The methods of Figs. 1-4, and the system of Fig. 5, may be implemented in hardware or software, or a mixture of both (for example, as firmware running on a hardware device). To the extent that an embodiment is implemented partly or wholly in software, the functional steps illustrated in the process flowcharts may be performed by suitably programmed physical computing devices, such as one or more central processing units (CPUs) or graphics processing units (GPUs). Each process - and its individual component steps as illustrated in the flowcharts - may be performed by the same or different computing devices. According to embodiments, a computer-readable storage medium stores a computer program comprising computer program code configured to cause one or more physical computing devices to carry out an encoding or decoding method as described above when the program is run on the one or more physical computing devices.

Storage media may include volatile and non-volatile computer memory such as RAM, PROM, EPROM, and EEPROM, optical discs (like CD, DVD, BD), magnetic storage media (like hard discs and tapes). Various storage media may be fixed within a computing device or may be transportable, such that the one or more programs stored thereon can be loaded into a processor.

To the extent that an embodiment is implemented partly or wholly in hardware, the blocks shown in the block diagrams of Fig. 6 may be separate physical components, or logical subdivisions of single physical components, or may be all implemented in an integrated manner in one physical component. The functions of one block shown in the drawings may be divided between multiple components in an implementation, or the functions of multiple blocks shown in the drawings may be combined in single components in an implementation. Hardware components suitable for use in embodiments of the present invention include, but are not limited to, conventional microprocessors, application specific integrated circuits (ASICs), and field-programmable gate arrays (FPGAs). One or more blocks may be implemented as a combination of dedicated hardware to perform some functions and one or more programmed microprocessors and associated circuitry to perform other functions.

A single processor or other unit may fulfil the functions of several items recited in the claims. The mere fact that certain measures are recited in mutually different dependent claims does not indicate that a combination of these measures cannot be used to advantage. If a computer program is discussed above, it may be stored/distributed on a suitable medium, such as an optical storage medium or a solid-state medium supplied together with or as part of other hardware, but may also be distributed in other forms, such as via the Internet or other wired or wireless telecommunication systems. If the term "adapted to" is used in the claims or description, it is noted the term "adapted to" is intended to be equivalent to the term "configured to". Any reference signs in the claims should not be construed as limiting the scope.

The flowchart and block diagrams in the Figures illustrate the architecture, functionality, and operation of possible implementations of systems, methods, and computer program products according to various embodiments of the present invention. In this regard, each block in the flowchart or block diagrams may represent a module, segment, or portion of instructions, which comprises one or more executable instructions for implementing the specified logical function(s). In some alternative implementations, the functions noted in the block may occur out of the order noted in the figures. For example, two blocks shown in succession may, in fact, be executed substantially concurrently, or the blocks may sometimes be executed in the reverse order, depending upon the functionality involved. It will also be noted that each block of the block diagrams and/or flowchart illustration, and combinations of blocks in the block diagrams and/or flowchart illustration, can be implemented by special purpose hardware-based systems that perform the specified functions or acts or carry out combinations of special purpose hardware and computer instructions, the architecture, functionality, and operation of possible implementations of systems, methods, and computer program products according to various embodiments of the present invention.

## Claims

1. A computer-implemented method (100) for controlling imaging modality of an oral imaging device, the method comprising:
determining the presence of an oral health feature (110) in a subject's mouth in view of a camera of the oral imaging device; and
controlling the oral imaging device (120) to switch from a first imaging modality to a second imaging modality based on the determined presence of an oral health feature.

2. The computer-implemented method of claim 1, wherein determining the presence of an oral health feature comprises analyzing an image (210) captured by the camera of the oral imaging device to detect the oral health feature.

3. The computer-implemented method of claim 1, wherein determining the presence of an oral health feature comprises:
capturing an image (310) of at least one oral feature of the subject using the camera of the oral imaging device, wherein an oral feature comprises a portion of at least one of: a tooth; gum; tongue; and a lip;
analyzing the captured image to determine the location of the camera (315); and
determining the presence of an oral health feature (318) in view of the camera based on the determined location and oral health data describing the presence and location of at least one oral health feature in the mouth of the subject.

4. The computer-implemented method of any prior claim, further comprising:
predicting a future location of the camera (440) at a future time point based on a motion of the camera and a present location of the camera;
determining the presence of an oral health feature at the predicted future location of the camera (450) based on the predicted future location and oral health data describing the presence and location of at least one oral health feature in the mouth of the subject; and
controlling the oral imaging device (460) to switch from a first imaging modality to a second imaging modality based on the determined presence of an oral health feature at the predicted future location of the camera.

5. The computer-implemented method of claim 4, wherein controlling the oral imaging device comprises controlling the oral imaging device to switch from a first imaging modality to a second imaging modality such that the oral imaging device will be in the second imaging modality at the future time point.

6. The computer-implemented method of claim 4 or 5, further comprising determining a motion of the camera (430) based on data from an inertial measurement sensor associated with the oral imaging device.

7. The computer-implemented method of any of claims 4 to 6, further comprising determining a motion of the camera (430) based on a captured series of images of at least one oral feature of the subject, wherein an oral feature comprises a portion of at least one of: a tooth; gum; tongue; and a lip.

8. The computer-implemented method of any of claims 3 to 7, wherein the oral health data further describes the presence and location of at least one predicted oral health feature in the mouth of the subject.

9. The computer-implemented method of any prior claim, wherein determining the presence of an oral health feature is further based on a demographic parameter of the subject, wherein a demographic parameter comprises at least one of: age; gender; race; ethnicity; weight; height; BMI; and geographic location.

10. The computer-implemented method of any prior claim, wherein an oral health feature comprises at least one of: a cavity; a crown; a bridge; a filling; plaque; tartar; gingivitis; periodontitis; tooth erosion; an abscessed tooth; malocclusion; a denture; a dental implant; braces; an impacted tooth; and a tumor.

11. The computer-implemented method of any prior claim, wherein the second imaging modality comprises at least one of: white light; quantitative light-induced fluorescence; fluorescence imaging with reflectance enhancement; and infrared light.

12. The computer-implemented of any prior claim, further comprising selecting the second imaging modality from a plurality of available imaging modalities based on the type of oral health feature determined to be present.

13. The computer-implemented method of any prior claim, wherein determining the presence of an oral health feature comprises using a machine-learning model trained to determine the presence of an oral health feature based on at least one image captured by the camera of the oral imaging device.

14. A computer program comprising code for implementing the method of any preceding claim when said program is run on a processor arrangement.

15. A system (500) for controlling imaging modality of an oral imaging device, comprising:
a processor arrangement (520) configured to:
determine the presence of an oral health feature in a subject's mouth in view of a camera of the oral imaging device; and
control the oral imaging device to switch from a first imaging modality to a second imaging modality based on the determined presence of an oral health feature.
